# EUROPEAN PATENT APPLICATION

(11) **EP 1 260 585 A1**
(43) Date of publication of application: **27.11.2002**
(21) Application number: 01906253.8
(22) Date of filing: 23.02.2001
(51) Int. Cl.: C12N 15/12, C07K 14/47, C07K 16/18

(54) **NOVEL PROTEIN AND GENE ENCODING THE SAME**

(30) Priority: 25.02.2000 JP 2000048727
(71) Applicant: DAIICHI PHARMACEUTICAL CO., LTD., Chuo-ku, Tokyo 103-8234 (JP)
(72) Inventor: SUZUKI, Makoto, Nakano-ku, Tokyo 165-0031 (JP); ISHIBASHI, Ken'ichi, Suginami-ku, Tokyo 167-0031 (JP)
(74) Representative: Wakerley, Helen Rachael
(86) International application number: JP0101354
(87) International publication number: WO01062915

(57) **Abstract**

A novel mechanosensitive channel protein derived from mouse or humans which is expressed specifically in the kidney and has the function of non-selectively transporting cation into cells in response to a mechanical stimulus; a DNA encoding these proteins; a method of screening cation channel activity activators or inhibitors with these proteins; and antibodies to these proteins. The above-described proteins are useful as a diagnostic agent or a therapeutic agent for diseases based on abnormalities in the cation channel, such as hypertension or diabetes, or as a tool for screening chemicals for preventing and/or treating these diseases.

SEQUENCE LISTING

<110> SNOW BRAND MILK PRODUCTS CO., LTD.

<120> NOVEL PROTEIN AND DNA CORDING THEREOF

<130> SNOW-135

<150> JP 48727-2000
<151> 2000-2-25

<160> 10

<170> PatentIn Ver. 2.1

<210> 1
<211> 871
<212> PRT
<213> MOUSE

<400> 1
Met Ala Asp Pro Gly Asp Gly Pro Arg Ala Ala Pro Gly Glu Val Ala
1               5                   10                  15
Glu Pro Pro Gly Asp Glu Ser Gly Thr Ser Gly Gly Glu Ala Phe Pro
            20                  25                  30
Leu Ser Ser Leu Ala Asn Leu Phe Glu Gly Glu Glu Gly Ser Ser Ser
        35                  40                  45
Leu Ser Pro Val Asp Ala Ser Arg Pro Ala Gly Pro Gly Asp Gly Arg
    50                  55                  60
Pro Asn Leu Arg Met Lys Phe Gln Gly Ala Phe Arg Lys Gly Val Pro
65                  70                  75                  80
Asn Pro Ile Asp Leu Leu Glu Ser Thr Leu Tyr Glu Ser Ser Val Val
                85                  90                  95
Pro Gly Pro Lys Lys Ala Pro Met Asp Ser Leu Phe Asp Tyr Gly Thr
            100                 105                 110
Tyr Arg His His Pro Ser Asp Asn Lys Arg Trp Arg Arg Lys Val Val

115                 120                 125
Glu Lys Gln Pro Gln Ser Pro Lys Thr Pro Ala Pro Gln Pro Pro Pro
    130                 135                 140
Ile Leu Lys Val Phe Asn Arg Pro Ile Leu Phe Asp Ile Val Ser Arg
145                 150                 155                 160
Gly Ser Thr Ala Asp Leu Asp Gly Leu Leu Ser Phe Leu Leu Thr His
                165                 170                 175
Lys Lys Arg Leu Thr Asp Glu Glu Phe Arg Glu Pro Ser Thr Gly Lys
            180                 185                 190
Thr Cys Leu Pro Lys Ala Leu Leu Asn Leu Ser Asn Gly Arg Asn Asp
        195                 200                 205
Thr Leu Gln Val Leu Leu Asp Ile Ala Glu Arg Thr Gly Asn Met Arg
    210                 215                 220
Glu Phe Ile Asn Ser Pro Phe Arg Asp Ile Tyr Tyr Arg Gly Gln Thr
225                 230                 235                 240
Ser Leu His Ile Ala Ile Glu Arg Arg Cys Lys His Tyr Val Glu Leu
                245                 250                 255
Leu Val Ala Gln Gly Ala Asp Val His Ala Gln Ala Arg Gly Arg Phe
            260                 265                 270
Phe Gln Pro Lys Asp Glu Gly Gly Tyr Phe Tyr Phe Gly Glu Leu Pro
        275                 280                 285
Leu Ser Leu Ala Ala Cys Thr Asn Gln Pro His Ile Val Asn Tyr Leu
    290                 295                 300
Thr Glu Asn Pro His Lys Lys Ala Asp Met Arg Arg Gln Asp Ser Arg
305                 310                 315                 320
Gly Asn Thr Val Leu His Ala Leu Val Ala Ile Ala Asp Asn Thr Arg
                325                 330                 335
Glu Asn Thr Lys Phe Val Thr Lys Met Tyr Asp Leu Leu Leu Leu Lys
            340                 345                 350
Cys Ser Arg Leu Phe Pro Asp Ser Asn Leu Glu Thr Val Leu Asn Asn
        355                 360                 365
Asp Gly Leu Ser Pro Leu Met Met Ala Ala Lys Thr Gly Lys Ile Gly
    370                 375                 380
Val Phe Gln His Ile Ile Arg Arg Glu Val Thr Asp Glu Asp Thr Arg
385                 390                 395                 400
His Leu Ser Arg Lys Phe Lys Asp Trp Ala Tyr Gly Pro Val Tyr Ser

                405                 410                 415
Ser Leu Tyr Asp Leu Ser Ser Leu Asp Thr Cys Gly Glu Glu Val Ser
            420                 425                 430
Val Leu Glu Ile Leu Val Tyr Asn Ser Lys Ile Glu Asn Arg His Glu
        435                 440                 445
Met Leu Ala Val Glu Pro Ile Asn Glu Leu Leu Arg Asp Lys Trp Arg
    450                 455                 460
Lys Phe Gly Ala Val Ser Phe Tyr Ile Asn Val Val Ser Tyr Leu Cys
465                 470                 475                 480
Ala Met Val Ile Phe Thr Leu Thr Ala Tyr Tyr Gln Pro Leu Glu Gly
                485                 490                 495
Thr Pro Pro Tyr Pro Tyr Arg Thr Thr Val Asp Tyr Leu Arg Leu Ala
            500                 505                 510
Gly Glu Val Ile Thr Leu Phe Thr Gly Val Leu Phe Phe Phe Thr Ser
        515                 520                 525
Ile Lys Asp Leu Phe Thr Lys Lys Cys Pro Gly Val Asn Ser Leu Phe
    530                 535                 540
Val Asp Gly Ser Phe Gln Leu Leu Tyr Phe Ile Tyr Ser Val Leu Val
545                 550                 555                 560
Val Val Ser Ala Ala Leu Tyr Leu Ala Gly Ile Glu Ala Tyr Leu Ala
                565                 570                 575
Val Met Val Phe Ala Leu Val Leu Gly Trp Met Asn Ala Leu Tyr Phe
            580                 585                 590
Thr Arg Gly Leu Lys Leu Thr Gly Thr Tyr Ser Ile Met Ile Gln Lys
        595                 600                 605
Ile Leu Phe Lys Asp Leu Phe Arg Phe Leu Leu Val Tyr Leu Leu Phe
    610                 615                 620
Met Ile Gly Tyr Ala Ser Ala Leu Val Thr Leu Leu Asn Pro Cys Thr
625                 630                 635                 640
Asn Met Lys Val Cys Asp Glu Asp Gln Ser Asn Cys Thr Val Pro Thr
                645                 650                 655
Tyr Pro Ala Cys Arg Asp Ser Glu Thr Phe Ser Ala Phe Leu Leu Asp
            660                 665                 670
Leu Phe Lys Leu Thr Ile Gly Met Gly Asp Leu Glu Met Leu Ser Ser
        675                 680                 685
Ala Lys Tyr Pro Val Val Phe Ile Leu Leu Leu Val Thr Tyr Ile Ile

690                 695                 700
Leu Thr Phe Val Leu Leu Leu Asn Met Leu Ile Ala Leu Met Gly Glu
705                 710                 715                 720
Thr Val Gly Gln Val Ser Lys Glu Ser Lys His Ile Trp Lys Leu Gln
                725                 730                 735
Trp Ala Thr Thr Ile Leu Asp Ile Glu Arg Ser Phe Pro Val Phe Leu
            740                 745                 750
Arg Lys Ala Phe Arg Ser Gly Glu Met Val Thr Val Gly Lys Ser Ser
        755                 760                 765
Asp Gly Thr Pro Asp Arg Arg Trp Cys Phe Arg Val Asp Glu Val Ser
    770                 775                 780
Trp Ser His Trp Asn Gln Asn Leu Gly Ile Ile Asn Glu Asp Pro Gly
785                 790                 795                 800
Lys Ser Glu Ile Tyr Gln Tyr Tyr Gly Phe Ser His Thr Val Gly Arg
                805                 810                 815
Leu Arg Arg Asp Arg Trp Ser Ser Val Val Pro Arg Val Val Glu Leu
            820                 825                 830
Asn Lys Asn Ser Ser Ala Asp Glu Val Val Val Pro Leu Asp Asn Leu
        835                 840                 845
Gly Asn Pro Asn Cys Asp Gly His Gln Gln Gly Tyr Ala Pro Lys Trp
    850                 855                 860
Arg Thr Asp Asp Ala Pro Leu
865                 870

<210> 2
<211> 2616
<212> DNA
<213> MOUSE

<400> 2
atggcagatc ctggtgatgg tccccgtgca gcgcctgggg aggtggctga gccccctgga 60
gatgagagtg gtacctctgg tggggaggcc ttcccctctc cttccctggc caatctgttt 120
gagggggagg aaggctcctc ttctctttcc ccggtggatg ctagccgccc tgctggccct 180
ggcgatggac gtccaaacct gcgtatgaag ttccagggcg ctttccgcaa gggggttccc 240
aaccccattg acctgttgga gtccaccctg tacgagtcct cagtagtgcc tgggcccaag 300
aaagcgccca tggattcctt gttcgactac ggcacttacc gtcaccaccc cagtgacaac 360

aagagatgga ggagaaaggt cgtggagaag cagccacaga gcccccaaac tcctgcaccc 420
cagccacccc ccatcctcaa agtcttcaat cggcccatcc tctttgacat tgtgtcccgg 480
ggctccactg cggacctaga tggactgctc tccttcttgt tgacccacaa gaagcgcctg 540
actgatgagg agttccggga gccgtccacg gggaagacct gcctgcccaa ggcgctgctg 600
aacctaagca acgggcgcaa cgacaccctc caggtgttgc tggacattgc ggagcgcacc 660
ggcaacatgc gtgaattcat caactcgccc ttcagagaca tctactaccg aggccagaca 720
tccctgcaca ttgccatcga acggcgctgc aagcactacg tggagctgct ggtggcccag 780
ggagccgacg tgcacgccca ggcccgcggc cgcttcttcc agcccaagga tgagggaggc 840
tacttctact ttggggagct gcccttgtcc ctggcagcct gcaccaacca gccgcacatc 900
gtcaactacc tgacagagaa ccctcacaag aaagctgaca tgaggcgaca ggactcgagg 960
gggaacacgg tgctgcacgc gctggtggcc atcgccgaca acacccgaga gaacaccaag 1020
tttgtcacca agatgtacga cctgctgctt ctcaagtgtt cacgcctctt ccccgacagc 1080
aacctggaga cagttctcaa caatgatggc ctttcgcctc tcatgatggc tgccaagaca 1140
ggcaagatcg gggtctttca gcacatcatc cgacgtgagg tgacagatga ggacacccgg 1200
catctgtctc gcaagttcaa ggactgggcc tatgggcctg tgtattcttc tctctacgac 1260
ctctcctccc tggacacatg cggggaggag gtgtccgtgc tggagatcct ggtgtacaac 1320
agcaagatcg agaaccgcca tgagatgctg gctgtagagc ccattaacga actgttgaga 1380
gacaagtggc gtaagtttgg ggctgtgtcc ttctacatca acgtggtctc ctatctgtgt 1440
gccatggtca tcttcaccct caccgcctac tatcagccac tggagggcac gccaccctac 1500
ccttaccgga ccacagtgga ctacctgagg ctggctggcg aggtcatcac gctcttcaca 1560
ggagtcctgt tcttctttac cagtatcaaa gacttgttca cgaagaaatg ccctggagtg 1620
aattctctct tcgtcgatgg ctccttccag ttactctact tcatctactc tgtgctggtg 1680
gttgtctctg cggcgctcta cctggctggg atcgaggcct acctggctgt gatggtcttt 1740
gccctggtcc tgggctggat gaatgcgctg tacttcacgc gcgggttgaa gctgacgggg 1800
acctacagca tcatgattca gaagatcctc ttcaaagacc tcttccgctt cctgcttgtg 1860
tacctgctct tcatgatcgg ctatgcctca gccctggtca ccctcctgaa tccgtgcacc 1920
aacatgaagg tctgtgacga ggaccagagc aactgcacgg tgcccacgta tcctgcgtgc 1980
cgcgacagcg agaccttcag cgccttcctc ctggacctct tcaagctcac catcggcatg 2040
ggagacctgg agatgctgag cagcgccaag taccccgtgg tcttcatcct cctgctggtc 2100
acctacatca tcctcacctt cgtgctcctg ttgaacatgc ttatcgccct catgggtgag 2160
accgtgggcc aggtgtccaa ggagagcaag cacatctgga agttgcagtg ggccaccacc 2220
atcctggaca tcgagcgttc cttccctgtg ttcctgagga aggccttccg ctccggagag 2280
atggtgactg tgggcaagag ctcagatggc actccggacc gcaggtggtg cttcagggtg 2340
gacgaggtga gctggtctca ctggaaccag aacttgggca tcattaacga ggaccctggc 2400
aagagtgaaa tctaccagta ctatggcttc tcccacaccg tggggcgcct tcgtagggat 2460
cgttggtcct cggtggtgcc ccgcgtagtg gagctgaaca agaactcaag cgcagatgaa 2520

gtggtggtac ccctggataa cctagggaac cccaactgtg acggccacca gcagggctac 2580
gctcccaagt ggaggacgga cgatgcccca ctgtag 2616

<210> 3
<211> 871
<212> PRT
<213> Homo sapiens

<400> 3
Met Ala Asp Ser Ser Glu Gly Pro Arg Ala Gly Pro Gly Glu Val Ala
1               5                   10                  15
Glu Leu Pro Gly Asp Glu Ser Gly Thr Pro Gly Gly Glu Ala Phe Pro
            20                  25                  30
Leu Ser Ser Leu Ala Asn Leu Phe Glu Gly Glu Asp Gly Ser Leu Ser
        35                  40                  45
Pro Ser Pro Ala Asp Ala Ser Arg Pro Ala Gly Pro Gly Asp Gly Arg
    50                  55                  60
Pro Asn Leu Arg Met Lys Phe Gln Gly Ala Phe Arg Lys Gly Val Pro
65                  70                  75                  80
Asn Pro Ile Asp Leu Leu Glu Ser Thr Leu Tyr Glu Ser Ser Val Val
                85                  90                  95
Pro Gly Pro Lys Lys Ala Pro Met Asp Ser Leu Phe Asp Tyr Gly Thr
            100                 105                 110
Tyr Arg His His Ser Ser Asp Asn Lys Arg Trp Arg Lys Lys Ile Ile
        115                 120                 125
Glu Lys Gln Pro Gln Ser Pro Lys Ala Pro Ala Pro Gln Pro Pro Pro
    130                 135                 140
Ile Leu Lys Val Phe Asn Arg Pro Ile Leu Phe Asp Ile Val Ser Arg
145                 150                 155                 160
Gly Ser Thr Ala Asp Leu Asp Gly Leu Leu Pro Phe Leu Leu Thr His
                165                 170                 175
Lys Lys Arg Leu Thr Asp Glu Glu Phe Arg Glu Pro Ser Thr Gly Lys
            180                 185                 190
Thr Cys Leu Pro Lys Ala Leu Leu Asn Leu Ser Asn Gly Arg Asn Asp
        195                 200                 205
Thr Ile Pro Val Leu Leu Asp Ile Ala Glu Arg Thr Gly Asn Met Arg

    210                 215                 220
Glu Phe Ile Asn Ser Pro Phe Arg Asp Ile Tyr Tyr Arg Gly Gln Thr
225                 230                 235                 240
Ala Leu His Ile Ala Ile Glu Arg Arg Cys Lys His Tyr Val Glu Leu
260 265 270
Leu Val Ala Gln Gly Ala Asp Val His Ala Gln Ala Arg Gly Arg Phe
            260                 265                 270
Phe Gln Pro Lys Asp Glu Gly Gly Tyr Phe Tyr Phe Gly Glu Leu Pro
        275                 280                 285
Leu Ser Leu Ala Ala Cys Thr Asn Gln Pro His Ile Val Asn Tyr Leu
    290                 295                 300
Thr Glu Asn Pro His Lys Lys Ala Asp Met Arg Arg Gln Asp Ser Arg
305                 310                 315                 320
Gly Asn Thr Val Leu His Ala Leu Val Ala Ile Ala Asp Asn Thr Arg
                325                 330                 335
Glu Asn Thr Lys Phe Val Thr Lys Met Tyr Asp Leu Leu Leu Leu Lys
            340                 345                 350
Cys Ala Arg Leu Phe Pro Asp Ser Asn Leu Glu Ala Val Leu Asn Asn
        355                 360                 365
Asp Gly Leu Ser Pro Leu Met Met Ala Ala Lys Thr Gly Lys Ile Gly
    370                 375                 380
Val Phe Gln His Ile Ile Arg Arg Glu Val Thr Asp Glu Asp Thr Arg
385                 390                 395                 400
His Leu Ser Arg Lys Phe Lys Asp Trp Ala Tyr Gly Pro Val Tyr Ser
                405                 410                 415
Ser Leu Tyr Asp Leu Ser Ser Leu Asp Thr Cys Gly Glu Glu Ala Ser
            420                 425                 430
Val Leu Glu Ile Leu Val Tyr Asn Ser Lys Ile Glu Asn Arg His Glu
        435                 440                 445
Met Leu Ala Val Glu Pro Ile Asn Glu Leu Leu Arg Asp Lys Trp Arg
    450                 455                 460
Lys Phe Gly Ala Val Ser Phe Tyr Ile Asn Val Val Ser Tyr Leu Cys
465                 470                 475                 480
Ala Met Val Ile Phe Thr Leu Thr Ala Tyr Tyr Gln Pro Leu Glu Gly
                485                 490                 495
Thr Val Pro Tyr Pro Tyr Arg Thr Thr Val Asp Tyr Leu Arg Leu Ala

            500                 505                 510
Gly Glu Val Ile Thr Leu Phe Thr Gly Val Leu Phe Phe Phe Thr Asn
        515                 520                 525
Ile Lys Asp Leu Phe Met Lys Lys Cys Pro Gly Val Asn Ser Leu Phe
    530                 535                 540
Ile Asp Gly Ser Phe Gln Leu Leu Ser Phe Ile Tyr Ser Val Leu Val
545                 550                 555                 560
Ile Val Ser Ala Ala Leu Tyr Leu Ala Gly Ile Glu Ala Tyr Leu Ala
                565                 570                 575
Val Met Val Phe Ala Leu Val Leu Gly Trp Met Asn Ala Leu Tyr Phe
            580                 585                 590
Thr Arg Gly Leu Lys Leu Thr Gly Thr Tyr Ser Ile Met Ile Gln Lys
        595                 600                 605
Val Leu Phe Lys Asp Leu Phe Arg Phe Leu Leu Val Tyr Leu Leu Phe
    610                 615                 620
Met Ile Gly Tyr Ala Ser Ala Leu Val Ser Leu Leu Asn Pro Cys Ala
625                 630                 635                 640
Asn Met Lys Val Cys Asn Glu Asp Gln Thr Asn Cys Thr Val Pro Thr
                645                 650                 655
Tyr Pro Ser Cys Arg Asp Ser Glu Thr Phe Ser Thr Phe Leu Leu Asp
            660                 665                 670
Leu Phe Lys Leu Thr Ile Gly Met Gly Asp Leu Glu Met Leu Ser Ser
        675                 680                 685
Thr Lys Tyr Pro Val Val Phe Ile Ile Leu Leu Val Thr Tyr Ile Ile
    690                 695                 700
Leu Thr Phe Val Leu Leu Leu Asn Met Leu Ile Ala Leu Met Gly Glu
705                 710                 715                 720
Thr Val Gly Gln Val Ser Lys Glu Ser Lys His Ile Trp Lys Leu Gln
                725                 730                 735
Trp Ala Thr Thr Ile Leu Asp Ile Glu Arg Ser Phe Pro Val Phe Leu
            740                 745                 750
Arg Lys Ala Phe Arg Ser Gly Glu Met Val Thr Val Gly Lys Ser Ser
        755                 760                 765
Asp Gly Thr Pro Asp Arg Arg Trp Cys Phe Arg Val Asn Glu Val Asn
    770                 775                 780
Trp Ser His Trp Asn Gln Asn Leu Gly Ile Ile Asn Glu Asp Pro Gly

785                 790                 795                 800
Lys Asn Glu Thr Tyr Gln Tyr Tyr Gly Phe Ser His Thr Val Gly Arg
                805                 810                 815
Leu Arg Met Asp Arg Trp Ser Ser Val Val Pro Arg Val Val Glu Leu
            820                 825                 830
Asn Lys Asn Ser Asn Pro Asp Glu Val Val Val Pro Leu Asp Ser Met
        835                 840                 845
Gly Asn Pro Arg Cys Asp Gly His Gln Gln Gly Tyr Pro Arg Lys Trp
    850                 855                 860
Arg Thr Asp Asp Ala Pro Leu
865                 870

<210> 4
<211> 2616
<212> DNA
<213> Homo sapiens

<400> 4
atggcggatt ccagcgaagg cccccgcgcg gggcccgggg aggtggctga gctccccggg 60
gatgagagtg gcaccccagg tggggaggct tttcctctct cctccctggc caatctgttt 120
gagggggagg atggctccct ttcgccctca ccggctgatg ccagtcgccc tgctggccca 180
ggcgatgggc gaccaaatct gcgcatgaag ttccagggcg ccttccgcaa gggggtgccc 240
aaccccatcg atctgctgga gtccacccta tatgagtcct cggtggtgcc tgggcccaag 300
aaagcaccca tggactcact gtttgactac ggcacctatc gtcaccactc cagtgacaac 360
aagaggtgga ggaagaagat catagagaag cagccgcaga gccccaaagc ccctgcccct 420
cagccgcccc ccatcctcaa agtcttcaac cggcctatcc tctttgacat cgtgtcccgg 480
ggctccactg ctgacctgga cgggctgctc ccattcttgc tgacccacaa gaaacgccta 540
actgatgagg agtttagaga gccatctacg gggaagacct gcctgcccaa ggccttgctg 600
aacctgagca atggccgcaa cgacaccatc cctgtgctgc tggacatcgc ggagcgcacc 660
ggcaacatga gggagttcat taactcgccc ttccgtgaca tctactatcg aggtcagaca 720
gccctgcaca tcgccattga gcgtcgctgc aaacactacg tggaacttct cgtggcccag 780
ggagctgatg tccacgccca ggcccgtggg cgcttcttcc agcccaagga tgaggggggc 840
tacttctact ttggtgagct gcccctgtcg ctggctgcct gcaccaacca gccccacatt 900
gtcaactacc tgacggagaa cccccacaag aaggcggaca tgcggcgcca ggactcgcga 960
ggcaacacag tgctgcatgc gctggtggcc attgctgaca acacccgtga gaacaccaag 1020
tttgttacca agatgtacga cctgctgctg ctcaagtgtg cccgcctctt ccccgacagc 1080

aacctggagg ccgtgctcaa caacgacggc ctctcgcccc tcatgatggc tgccaagacg 1140
ggcaagattg gggtgtttca gcacatcatc cggcgggagg tgacggatga ggacacacgg 1200
cacctgtccc gcaagttcaa ggactgggcc tatgggccag tgtattcctc gctttatgac 1260
ctctcctccc tggacacgtg tggggaagag gcctccgtgc tggagatcct ggtgtacaac 1320
agcaagattg agaaccgcca cgagatgctg gctgtggagc ccatcaatga actgctgcgg 1380
gacaagtggc gcaagttcgg ggccgtctcc ttctacatca acgtggtctc ctacctgtgt 1440
gccatggtca tcttcactct caccgcctac taccagccgc tggagggcac agtgccgtac 1500
ccttaccgca ccacggtgga ctacctgcgg ctggctggcg aggtcattac gctcttcact 1560
ggggtcctgt tcttcttcac caacatcaaa gacttgttca tgaagaaatg ccctggagtg 1620
aattctctct tcattgatgg ctccttccag ctgctcagct tcatctactc tgtcctggtg 1680
atcgtctcag cagccctcta cctggcaggg atcgaggcct acctggccgt gatggtcttt 1740
gccctggtcc tgggctggat gaatgccctt tacttcaccc gtgggctgaa gctgacgggg 1800
acctatagca tcatgatcca gaaggtactc ttcaaggacc ttttccgatt cctgctcgtc 1860
tacttgctct tcatgatcgg ctacgcttca gccctggtct ccctcctgaa cccgtgtgcc 1920
aacatgaagg tgtgcaatga ggaccagacc aactgcacag tgcccactta cccctcgtgc 1980
cgtgacagcg agaccttcag caccttcctc ctggacctgt ttaagctgac catcggcatg 2040
ggcgacctgg agatgctgag cagcaccaag taccccgtgg tcttcatcat cctgctggtg 2100
acctacatca tcctcacctt tgtgctgctc ctcaacatgc tcattgccct catgggcgag 2160
acagtgggcc aggtctccaa ggagagcaag cacatctgga agctgcagtg ggccaccacc 2220
atcctggaca ttgagcgctc cttccccgta ttcctgagga aggccttccg ctctggggag 2280
atggtcaccg tgggcaagag ctcggacggc actcctgacc gcaggtggtg cttcagggtg 2340
aatgaggtga actggtctca ctggaaccag aacttgggca tcatcaacga ggacccgggc 2400
aagaatgaga cctaccagta ttatggcttc tcgcataccg tgggccgcct ccgaatggat 2460
cgctggtcct cggtggtacc ccgcgtggtg gaactgaaca agaactcgaa cccggacgag 2520
gtggtggtgc ctctggacag catggggaac ccccgctgcg atggccacca gcagggttac 2580
ccccgcaagt ggaggactga tgacgccccg ctctag 2616

<210> 5
<211> 19
<212> DNA
<213> Artificial Sequence

<220>
<223> Description of Artificial Sequence: Synthesized DNA

<400> 5

gagagaacac caagtttgt 19

<210> 6
<211> 26
<212> DNA
<213> Artificial Sequence

<220>
<223> Description of Artificial Sequence: Synthesized DNA

<400> 6
cagcggcccc aatctcttca aagtac 26

<210> 7
<211> 20
<212> DNA
<213> Artificial Sequence

<220>
<223> Description of Artificial Sequence: Synthesized DNA

<400> 7
ctacagccag catctcatgg 20

<210> 8
<211> 30
<212> DNA
<213> Artificial Sequence

<220>
<223> Description of Artificial Sequence: Synthesized DNA

<400> 8
tcagggtcca gtatggcaga tcctggtgat 30

<210> 9

<211> 22
<212> DNA
<213> Artificial Sequence

<220>
<223> Description of Artificial Sequence: Synthesized DNA

<400> 9
gttaatgggc tctacagcca gc 22

<210> 10
<211>
<212> PRT
<213> Homo sapiens

<400> 10
Pro Leu Asp Asn Leu Gly Asn Pro Asn Cys
 1               5                   10

## Description

### Field of the Invention

The present invention relates to a novel mechanosensitive channel protein derived from mice or humans which is expressed specifically in the kidney (SAC1 (mouse) and hSAC (human)) , DNA encoding said proteins , a method of screening cation channel activators or inhibitors using the proteins, and antibodies to the proteins.

The protein, DNA, and antibodies to the protein of the present invention are useful as a diagnostic agent or a therapeutic agent for diseases based on abnormalities in the cation channel such as hypertension or diabetes. They are also useful as a tool for screening chemicals for preventing and/or treating these diseases. In the present invention, mechanosensitive channel proteins such as SAC1, hSAC, and the like are generically referred to as SAC.

### Background Art

An ion channel is a protein with a pore structure which is located in biomembranes consisting of a lipid bilayer as a basic structure. The ion channel is classified according to the electrophysiological characteristics, i.e. gating, conductance, ion selectivity and the like. Ion channel molecules have multiple conformations. Namely, the ion channel molecules allow ions to permeate through in a certain state (open state) , but do not allow ions to permeate in another state (closed state). Such a change in the conformation is referred to as opening and closing of a gate (gating) . Three factors are known to control the gating: membrane potential, binding of low molecules (ligands) and membrane expansion.

There are many examples of voltage-dependent channels such as Na⁺ channel, Ca²⁺ channel, K⁺ channel, and the like. These channels have a molecular structure in which charges are gathered, called an electric sensor. Changes in the conformation are thought to occur by a force caused by an electric potential difference between the two sides the membrane. So ions can move through the pore. A ligand-sensitive channel is activated by binding of a specific ligand (agonist) from outside the cell. This is also called a receptor built-in channel. On the other hand, there is a channel whose opening and closing operations are controlled by binding of molecules with a low molecular weight, such as second messengers, existing inside the cells. An example is an IP₃ (inositol trisphosphate) receptor existing in cell membrane. A Ca²⁺ channel opens when IP₃ binds to the receptor at the cytoplasmic side. A mechanosensitive channel is an ion channel in which opening and closing of the gate is controlled by stretching of the membrane. Since most channels open the gate by stretching, these are also called "stretch sensitive" channels. The channel is thought to be involved in the osmotic pressure regulation and volume adjustment of cells . It is quite natural that the ion channel exists in a stretch receptor such as muscle spindles. The ion channels have been discovered in many kind of cells up to now (Morris C. E., J. Membrane Biol., 113, 93-107 (1990); Bear, C. E., Am. J. Physiol., 258, C421-8 (1990) ; Cemerik, D. & Sackin, H., Am. J. Physiol., 264, F697-714 (1993); Lansman, J. B., et al., Nature, 325, 811-3 (1987); Sackin, H., Am. J. Physiol., 253, F1253-62 (1987)).

In addition, it has been indicated that a K⁺ channel of mammals encoding a TWIK1 (tandem of P domains in a weak inward rectifier K⁺ channel) related TASK (TWIK-1 related acid-sensitive K⁺ channel) having four transmembrane domains possesses the characteristics of opening and closing by mechanical stimulus (Duprat, F. et al.,(1997) EMBO J., 16, 5464-71, Patel, A. J., et al.,(1998) EMBO J. 17, 4283-90).

The inventors of the present invention have conducted gene cloning based on the hypothesis that a vanilloid receptor sensitive to heat as a physical factor will be a mechanosensitive channel. As a result, the inventors have already presented a report on a cDNA similar to the vanilloid receptor (Caterina, M. J., et. al., (1997) Nature, 389,816-24) and encoding a nonselective cation channel (SIC) having an ankyrin repeat and six transmembrane domains and controlled by stretch (Suzuki, M., et. al., (1999) J. Biol. Chem. 274, 6330-5). Since a mechanosensitive channel can convert a mechanical stimulus into a Ca²⁺ influx, a stretch-activated (SA) nonselective cation channel is important.

### Disclosure of the Invention

In view of this situation, the inventors of the present invention have conducted an extensive investigation for a novel mechanosensitive channel protein. As a result, the inventors have found a protein expressed specifically in the kidney and having a function of non-selectively incorporating cations into cells in response to a mechanical stimulus, Namely a novel mechanosensitive channel protein. Accordingly, an object of the present invention is to provide a novel mechanosensitive channel protein derived from mice or humans expressed specifically in the kidney and possessing a function of non-selectively incorporating cations into cells in response to a mechanical stimulus (SAC1 (mouse) (Stretch Activated Channel protein 1) and hSAC (human) (human Stretch Activated Channel protein)), DNA encoding these proteins, a method of screening a cation channel activators or inhibitors using the proteins, and antibodies to the proteins.

The present invention relates to a novel mechanosensitive channel protein derived from mice or humans (SAC1 (mouse) and hSAC (human)) which is expressed specifically in the kidney and has a function of non-selectively incorporating cations into cells in response to a mechanical stimulus. The present invention also relates to DNA encoding the proteins. Furthermore, the present invention relates to a method of screening cation channel activators or inhibitors using these proteins. The present invention also relates to antibodies to these proteins. The proteins, the DNA, and the antibodies to the proteins can be used as a diagnostic agent or a therapeutic agent for diseases based on abnormalities in the cation channel, such as hypertension or diabetes, or as a tool for screening chemicals for preventing and/or treating these diseases.

Advanced technologies such as molecular biotechnology, cell biological technology, and electrophysiological technology are required in order to carry out the isolation, identification, and functional analysis of a novel membrane channel. The present inventors have isolated a cDNA of an SA cation channel (SAC1) from mouse kidney and have determined the amino acid sequence using these technologies. The cDNA belongs to a superfamily of vanilloid receptors and is localized in the uriniferous tubules of the kidney. The SAC1 was subjected to expression in a mammal cell line. Furthermore, a channel gene corresponding to that of humans was obtained using the SAC1 gene which is of mouse origin.

As a result of electrophysiological analysis, intracellular free calcium has been increased by mechanically stimulating transformed cells that express SAC1. This is proved to be a single channel activated by negative pressure, being blocked by Gd₃³⁺ and non-selective with respect to permeation of cations; the ionic permeability of SAC1 is of Na⁺>K⁺. The reversal potential of the whole cells shifted toward positive by inflating the cells. In addition, since a deletion mutant of the channel becomes unresponsive to stretch, it is suggested that the SAC1 may become an SA channel.

As mentioned above, the present invention relates to a novel mechanosensitive channel protein derived from mice or humans (SAC1 (mouse) and hSAC (human)) which is expressed specifically in kidney and has a function of non-selectively incorporating cations into cells in response to a mechanical stimulus, and to DNA encoding these proteins. The protein of the present invention can be obtained as follows. A mouse kidney cDNA library is constructed and amplified with a PCR method using suitable primers to obtain SAC1 cDNA. hSAC cDNA can then be obtained by cloning from a human kidney cDNA library using this SAC1 cDNA as a probe. The SAC1 derived from mice is a protein having 871 amino acid residues, whereas the hSAC derived from humans is also a protein having 871 amino acid residues. These exhibit 94.4% homology between the amino acid sequences thereof. This significant homology can be thought to indicate that SAC1 has common structures and functions in mammalian cells. The DNA encoding the hSAC has a length of about 18 Kb, is composed of 14 exons, and exists on chromosome 12q 24.1.

A mouse or human SAC protein can be obtained by inserting the obtained SAC gene derived from mouse or human in a suitable vector and transforming the host cells with the vector. As host cells, bacteria, yeast, animal cells, and the like can be used. Particularly, HeLa cells, Chinese hamster ovary (CHO) cells, or COS-7 cells are preferable as animal cells. In addition, a promoter such as a virus polyoma, adenovirus, cytomegalovirus, or simian virus 40 can be used for controlling an expression plasmid of cell line. Particularly, as a useful plasmid in expression system of animal cells, pCMV is preferably known (Thomsen, et al., PNAS, (1984) 81, 659). The resulting protein is a membrane-bound protein sensitive to mechanical stimulus and can be used as a diagnostic agent or a therapeutic agent for diseases based on abnormalities in the cation channel such as hypertension or diabetes, also used as a tool for screening chemicals for preventing and/or treating these diseases.

In addition, the proteins of the present invention or the fragments thereof are useful for diagnosis of defective hSAC by DNA hybridization. Mutants of hSAC are useful for research of hypertension and diabetes. Furthermore, fusion proteins can be easily prepared by connecting a nucleotide sequence encoding other proteins or synthetic polypeptides to 5' or 3' terminals of SAC DNA or its mutant using conventionally known technology. For example, the fusion protein may be prepared as a precursor protein, and caused to function when digested *in vitro* or *in vivo,* and possess selective distribution in a objective tissue and the like in addition to the inherent functions.

Moreover, the expressed proteins, mutants, or fragments thereof may immunize an animal to produce a polyclonal antibody. In addition, a monoclonal antibody can be prepared using hybridoma cells produced by fusion of lymphocytes obtained from an immunized animal and myeloma cells.

The proteins of the present invention or their mutants or analogues can be used as a diagnostic agent or therapeutic agent for diseases relating to channel proteins, or also for screening substances agonistically or antagonistically acting on SAC. Acquisition of the DNA sequence information on SAC has made it easy to prepare a partial DNA or RNA sequence. Since such a partial DNA sequence is capable of hybridizing to genes to be selected, the DNA can be used as a nucleic acid probe. Such a probe is useful for detecting cDNA sequences in various tissues. It is possible to obtain nucleic acids able to hybridize from various organisms and their tissues using the probe prepared by using the SAC. The resulting nucleic acids include a nucleic acid encoding a protein exhibiting a same isotype as SAC or a novel feature. In addition, the probe prepared can be used for gene diagnosis of diseases. It is possible to detect a disease gene by identifying the nucleotide sequence from a patient hybridized with the probe. A gene therapeutic agent used for substantial treatment can also be prepared. The nucleotide sequence of SAC and its mutant or derivatives thereof can be incorporated in a plasmid or stem cells and administered as an agent for gene therapy.

The protein of the present invention and the antibodies to the protein can be administered safely to human being and primate animals. The protein of the present invention can be prepared to a pharmaceutically acceptable formula and administered orally or non-orally. Example pharmaceutical compositions include compositions for injection, infusion, suppositories, nasal agents, buccals, percutaneous absorption agents, and the like. These compositions are formulated according to known pharmaceutical preparation methods using pharmaceutically acceptable carriers, vehicles, stabilizers, coloring agents, surfactants, and/or other additives, and made into objective formulations. In preparing compositions for injection, a pharmacologically effective amount of protein of the present invention may be mixed with a pharmaceutically acceptable vehicles, such as amino acids, saccharides, cellulose derivatives, other organic compounds and/or inorganic compounds.

In addition, in preparing a composition for injection using the antibodies of the present invention such vehicles and/or activating agents, a pH adjusting agent, buffering agent, stabilizer, solubilizing agent, and the like, may be optionally added according to conventional methods.

The DNA of the present invention can be used for gene therapy either by itself alone, by incorporating into a liposome, or by inserting into a vector for gene therapy such as retrovirus and adenovirus.

### Brief Description of the Drawings

Figure 1 (a) shows results of northern blotting analyses of SAC1 mRNA expression using the full length cDNA. RNA (2 µg) prepared from mouse tissues was applied to each lane.
Figure 1(b) shows a result of immunostaining of a kidney tissue. Stained areas are shown in white.
Figure 2 shows a result of the effect of SAC1 expression cells (GFP positive) on touch stress. In this figure, ○ indicates the fluorescence intensity at 380 nm, ▲ indicates the fluorescence intensity ratio (360 nm/380 nm), and ● indicates the fluorescence intensity at 360 nm.
Figure 3 shows results of response to stretch of SAC single channel, wherein
   (A) shows a typical result of SAC1 channel when negative pressure was applied stepwise,
   (B) shows the result when negative pressure was gradually applied, and
   (C) shows the relationship between pressure and Po when the pressure was applied to SAC1 with or without addition of GdCl₃. In the Figure, ○ indicates a control (pressure without the addition of GdCl₃) and Δ indicates pressure with the addition of 0.5 M GdCl₃, respectively.

### Preferred Embodiment of the Invention

### EXAMPLES

The present invention will now be described in more detail by way of examples, which are given for the purpose of explanation and should not be construed as limiting the present invention.

### Example 1

### Isolation of cDNA encoding SAC1

RNA was isolated by the guanidine thiocyanate method using Trizol (Gibco-BRL). mRNA was purified using a polyA column (Pharmacia). The cDNA library of mouse kidney was prepared using a Marathon cDNA construction kit (Clonetech Co.). The cDNA was amplified by PCR (30 cycles, one cycle consisting of 30 seconds at 94°C and 4 minutes at 70°C) using a primer set 1 (AA13f2 (SEQ ID No: 5 in Sequence Listing) and mA13end1 (SEQ ID No: 6)) and ExTaq polymerase (Takara, Ltd.). A nested primer (AA13r2 (SEQ ID No: 7)) was synthesized to determine the 5' terminal by the 5' RACE method.

Using the primer set 2 (A13st1 (SEQ ID No: 8) and AA13r1 (SEQ ID No: 9)) and ExTaq polymerase (Takara, Ltd.), PCR amplification was carried out under the same conditions as described above. Using each fragment obtained by PCR amplification using the two primer sets, the region containing the full length was amplified by PCR using primer set (A13st1 and mA13end1). SAC1 cDNA with a length of about 3.2 Kb was again cloned from the cDNA library. The cloned cDNA was ligated to a TA cloning vector (TOPO-XL; InVitrogen) and its *BamH*I-*Hind*III fragment was ligated to a mammalian cells expression vector (pCMV-SPORT; Gibco-BRL). Sequencing was carried out by an automatic sequencer (model 373-S; Applied Bioinstruments Inc.) using Thermo Sequenase dye terminator cycle sequencing premix. SAC1cDNA was a 2616 nucleotide encoding 871 amino acids. The sequences are shown as SEQ ID No: 1 (amino acid sequence) and SEQ ID No: 2 (nucleotide sequence) in Sequence Listing. The plasmid in which cDNA of SAC1 is inserted was designated pmSAC1 TOPO and originally deposited with the Biotechnology Laboratory, National Institute of Advanced Science and Technology, the Ministry of Economy, Trade and Industry (1-3, Higashi 1-Chome, Tsukuba-shi, Ibaraki-ken, Japan 305-8566) on November 30, 1999. The deposition was subject to a request to transfer to a deposition under the Budapest Treaty on November 1, 2000, and received as FERM BP-7345.

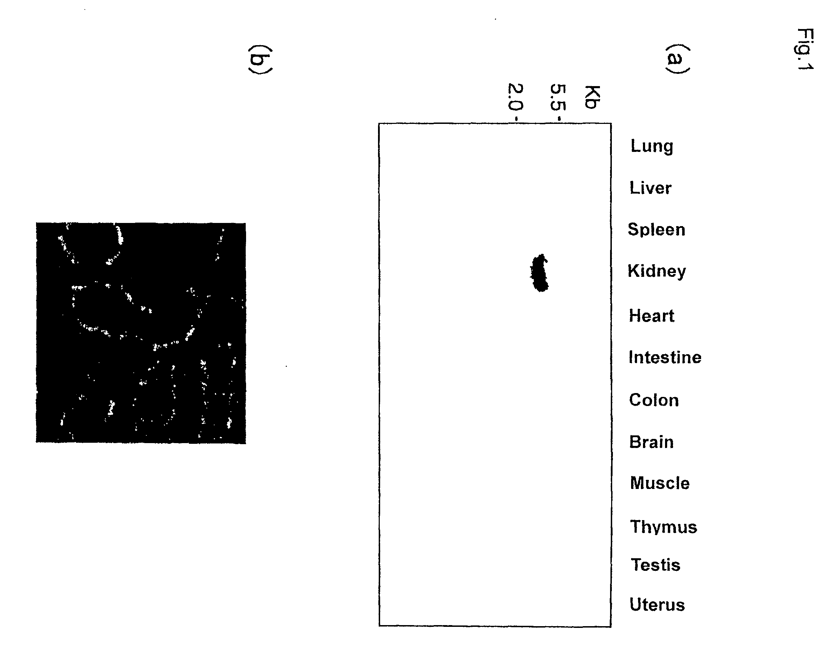

Next, expression of SAC1 in each tissue was confirmed by Northern hybridization (65°C) using SAC1 cDNA labeled with ³²P as a probe. As a result, SAC1 mRNA with a length of about 3 Kb was confirmed to have been expressed only in the kidney (Figure 1(a)).

In addition, to isolate cDNA encoding the human origin SAC (hSAC), a human kidney cDNA library was screened using ³²P-labeled SAC1 cDNA as a probe. The resulting clone was subcloned into vectors M13mp1, M13mp19 (Takara, Ltd.) and pGEM3Z (Promega Co.) to determine the nucleotide sequence in the same manner as above. The sequences are shown in SEQ ID No: 3 (amino acid sequence) and SEQ ID No: 4 (nucleotide sequence) in Sequence Listing.

### Example 2

### Antibody preparation and immunostaining

An antibody to the specific peptide in the C-terminal (844-853) of SAC1 protein (hereinafter referred to as "C-terminal peptide") (SEQ ID No: 10 in Sequence Listing) bound to polyethylene glycol was prepared. The C-terminal peptide of the mouse SAC1 protein was bound to the polyethylene glycol (PEG) in accordance with the method of Maeda et al. (Biochem. Biophys. Res. Comm. (1998) 248. 485-489). Briefly, one equivalent dicyclohexylcarbodiimide was added to oxidized PEG dissolved in dichloromethane (Wako Pure Chemicals Co., Ltd.) and the mixture was stirred at 0°C. Then, 10 equivalents of ethylenediamine were added and the mixture was stirred overnight. The product obtained after evaporating the solvent from the filtrate was dissolved in 50% acetic acid. Amino acid-type PEG (aaPEG) was purified by a Sephadex G-25 column and the N-terminal amino group was converted into an Fmoc group to prepare Fmoc-aaPEG. Fmoc-aaPEG and Boc-PLD (Pmc) NLGNPNC-OH were bound to a Rink resin (300 mg; Watanabe Chemical Co., Ltd.) in that order using the diisopropyl carbodiimide/1-hydroxybenzotriazole method (Konig and Geiger, Chem. Ber. (1970) 103, 788-798, 2024-2033). After removing the Fmoc group, the resin was treated with trifluoroacetic acid/ thioanisole/anisole/ ethanedithiol (94/2/2/2) for 3 hours to cleave out C-terminal peptide-aaPEG. The crude peptide precipitated by adding ether to the filtrate was purified using reverse phase HPLC, thereby obtaining 43 mg of C-terminal peptide-aaPEG.

Two NZW rabbits were immunized by intramuscular injection of 1 mg of the antigen (C-terminal peptide-aaPEG) emulsified with Freund's complete adjuvant. Thereafter, the rabbits were immunized in the same manner every two weeks using the same amount of antigen emulsified with Freund's incomplete adjuvant. During 4-8 weeks after the administration, the antibody titer of serum was measured by an ELISA method. The serum exhibiting titer of 10,000 times or more than that of the control serum was prepared. The antibody was purified from the serum by Protein A column (Pharmacia Co.), followed by affinity purification using a Proton kit (Multiple Peptide System Co.). After staining a fresh kidney slice with the antibody diluted to 500 fold, the protein was detected using an anti-rabbit IgG antibody (DAKO Co.) labeled with FITC. As a result, the basolateral membrane of proximal tubule was stained, it was thus confirmed that the SAC1 protein is localized (see Figure 1(b)).

### Example 3

### Expression of cDNA

The plasmid (pEGFP-N1; Clonetech Co.) expressing a green fluorescent protein was used as a marker for transfection. For the transient expression of protein, CHO cells were cultured in a HamF-12 culture medium (Gibco-BRL Co.) supplemented with 10% fetal bovine serum, 100 U/ml penicillin, and 100 µg/ml streptomycin. On the day before transformation, CHO cells (10⁵ cells) were seeded to a 35 mm dish in which a cover slip coated with rat tail collagen was placed. On the next day, SAC1 plasmid (1 µg) in pCMV-SPORT (Gibco-BRL Co.) and pEGFP-N1 (1 µg) were added per one transfection to a mixture of serum-free medium (97 µl) and FuGENE6 (Roche, 3 µl) which had been incubated for 5 minutes at room temperature. After incubation for 15 minutes at room temperature, the mixture was added to a dish containing 3 ml of serum-added medium. Cells grown on the cover slip were used as a patch clamp sample. A 10 % FCS-containing culture medium was used for the growth and transfection. The electrophysiological test was started after 24 hours and the fluorescence measurement was carried out 48 hours after the transfection.

### Example 4

### Fluorescence measurement of SAC1 expression cells

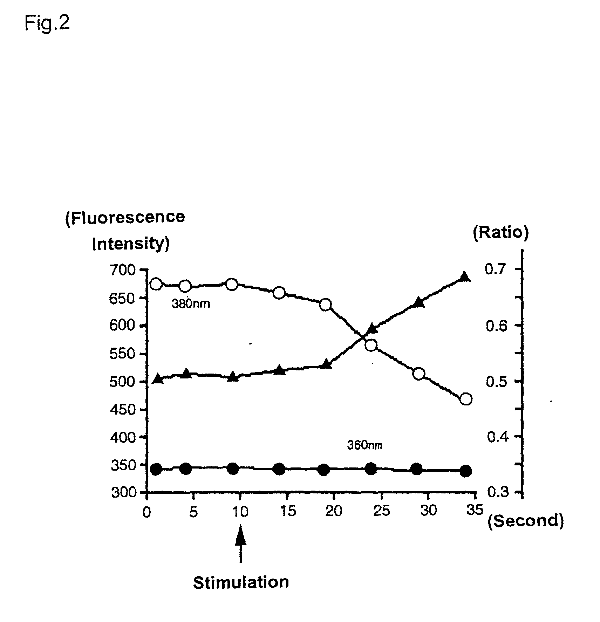

For 2-3 hours prior to start of the test, the transformed cells were incubated with 10 µM fura-2AM (Doj in Co.) dissolved in a serum-free culture medium. GFP fluorescence at 480 nm and fura-2 fluorescence at 360/380 nm were visualized by a manual exchange of dichroic Olympus system mirrors (Merlin Co.). Images for the fluorescence at 480 nm and fluorescence at 360/380 nm were obtained every two seconds. The fluorescence intensity of the objective area was calculated for analysis. Cells were bathed in a solution of 125 mM NaCl, 5 mM KCl, 1.2 mM MgSO₄, 1 mM Na₂HPO₄, 1 mM CaCl₂, and 3 mM HEPES (pH 7.4) at 34°C. To produce a mechanical stress directly to cells, they were touched by a glass pipette with a round tip using an electrically controlled micromanipulator (Model 5170; Eppendorf Co.) . The results obtained by inducing a mechanical stimulus to light cells (GFP positive) using the glass pipette are shown in Figure 2 and Table 1. The manifest change of the fura-2 fluorescence ratio in the SAC1 expression cells as compared with the control cells was observed, thus it was indicated that the proteins of the present invention function as channel proteins, clearly bringing about an alteration of [Ca²⁺]i (intracellular calcium ion concentration).

**TABLE 1**

| | no added Gd³⁺ | added Gd³⁺ |
|---|---|---|
| Control cells | 4.5 ± 3.8(8) | |
| SAC1 expression cells | 12.8 ± 1.4**(8) | 4.8 ± 1.4 (10) |
| (The number in parentheses is the number of experiments, "**" indicates 1% significant difference (p<0.01) compared with control, and "*" indicates 5% significant difference (p<0.05) compared with "no added Gd³⁺".) | | |

### Example 5

### Single channel analysis of SAC1 expression cells

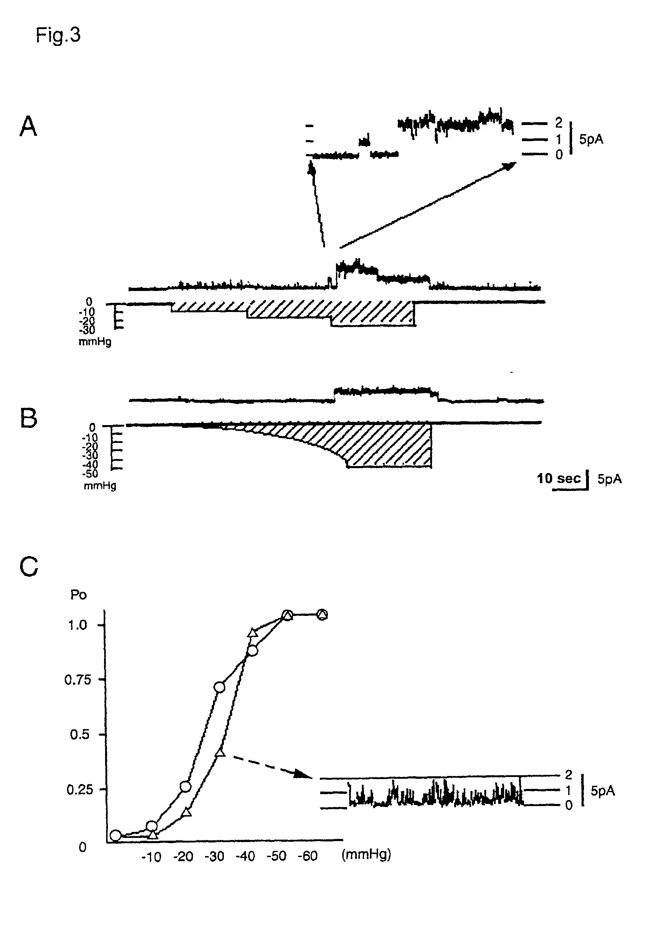

Patch clamp recordings were carried out according to the method described in a previous paper (Suzuki, M., Sato, J., Kutsuwada, K., Ooki, G. and Imai, M. (1999) J. Biol. Chem. 274, 6330-5). GFP positive cells were visualized by a fluorescence measurement with an emission of 490 nm (CAM 2000 system; Jusco Co., Ltd.). Cells were set on a mount under a vectorial flow of solution at a rate of 1 ml/ml. To control the temperature, the solution was heated to 34°C by a DC supply (Model LPD; Kikusui Electronic Co., Ltd.) prior to mounting. A solution of 140 mM NaCl, 5 mM KCl, 1.2 mM MgSO₄, 1 mM Na₂HPO₄, 1 mM CaCl₂, and 3 mM HEPES was used as a bath solution. In single channel analysis, the current was recorded with an EPC-7 patch clamp amplifier (List-Electronic Co., Ltd.) and stored on a DAT recorder (DAT-200; Sony Corp.) at 10 KHz. A 5 KHz filter was applied for the analysis. The records were sampled by Fetchex (Software Axon, version 6.0) to analyze the open probability. The data were analyzed using Igor ver. 2.01 and Patch Analysist ver. 1.21. A digital filter was used at 2 KHz to calculate the open probability by current distribution analysis. To analyze the open probability in the presence of GdCl₃, single channel amplitude was set for those without reagent and the mean open probability (Po) was determined by the records during 10 seconds . The pipette pressure was adjusted using a manometer and negative pressure was manually changed. To examine the channel opening, negative pressure was applied through a cell attaching pipette using a 150 mM NaCl solution. As a result, more than one half of the patches on expressed cells revealed channel activity by negative pressure. As shown in Figure 3A, the SAC1 channel abruptly opens at 30 mmHg negative pressure. The open probability suddenly reached the state of full opening. In order to confirm this phenomenon, the suction was gradually applied (Figure 3B). The channel suddenly opened as if there was a threshold for opening. The threshold was constant (33±2.3 mmHg) and reproducible. Current-voltage relationship was measured with pipettes filled with various solutions (sodium chloride, sodium gluconate and potassium chloride) to confirm that there was observed no significant changes. A single channel conductance was measured as slope conductance with cell-attach configuration. When sodium chloride was substituted with sodium gluconate or potassium chloride, there was almost no change in the parameters, thus it was clearly confirmed that the channel is non-selective with respect to cations. Accumulation of all-or-none openings of individual channels with different sensitivities to pressure, resulted in an S-shaped opening response to stretch. The mean open probability (Po) however did not fit to the Bolzmann's function of the pressure. The response was not affected when 0.5 µM GdCl₃ was added to the pipette, confirming that the protein possesses a function as a mechanosensitive channel protein (Figure 3C).

### Industrial Applicability

According to the present invention, novel mechanosensitive channel protein derived from mice or humans (SAC1 (mouse) and hSAC (human)) are provided. The proteins possess a function of being expressed specifically in the kidney and non-selectively incorporating cations into cells in response to mechanical stimulus. DNA encoding these proteins, a method of screening cation channel activators or inhibitors using said proteins and antibodies to the proteins are also provided.

The proteins, DNA, and antibodies to the proteins can be used as a diagnostic agent or a therapeutic agent for diseases based on abnormalities in the cation channel such as hypertension or diabetes or as a tool for screening chemicals for preventing and/or treating these diseases.

### Remarks to Deposited Biological Materials

a. Name and address of the organization in which the biological materials have been deposited:
   Name: The Biotechnology Laboratory, National Institute of Advanced Science and Technology, the Ministry of Economy, Trade and Industry
   Address: 1-1-3, Higashi, Tsukuba-shi, Ibaraki-ken, Japan (Postal Code: 305-3566).
b. Date of deposition: November 30, 1999 (Original deposition)
c. Number of deposition given by the deposition organization: FERM BP-7345

## Claims

1. A protein wherein said protein is expressed specifically in the kidney and has a function of transporting cations non-selectively into cells in response to a mechanical stimulus.

2. The protein according to claim 1, which is derived from mice.

3. The protein according to claim 2, having an amino acid sequence shown in SEQ ID No: 1 in the Sequence Listing.

4. A DNA encoding the amino acid sequence described in claim 3.

5. The DNA according to claim 4, having a nucleotide sequence shown in SEQ ID No: 2 in the Sequence Listing.

6. The protein according to claim 1, which is derived from humans.

7. The protein according to claim 6, having an amino acid sequence shown in SEQ ID No: 3 in the Sequence Listing.

8. A DNA encoding the amino acid sequence described in claim 7.

9. The DNA according to claim 8, having a nucleotide sequence shown in SEQ ID No: 4 in the Sequence Listing.

10. A protein according to claim 3 or 7, having an amino acid sequence in which one or more amino acids are deleted from, substituted in or added to the given sequence.

11. A DNA hybridizing with the DNA described in any one of claims 4, 5, 8, and 9.

12. A method of screening cation channel activators or inhibitors using the protein described in any one of claims 1, 2, 3, 6, and 7.

13. An antibody to the protein described in any one of claims 1, 2, 3, 6, and 7.

14. The antibody according to claim 13, wherein the antibody is a monoclonal antibody.
